(19) **Europäisches Patentamt**
**European Patent Office**
**Office européen des brevets**

(11) **EP 3 179 904 B1**

(12) **EUROPEAN PATENT SPECIFICATION**

(45) Date of publication and mention
of the grant of the patent:
**30.10.2019 Bulletin 2019/44**

(21) Application number: **15826858.1**

(22) Date of filing: **24.06.2015**

(51) Int Cl.:
*A61B 5/022* (2006.01)          *A61B 5/0245* (2006.01)
*A61B 5/0225* (2006.01)        *A61B 5/00* (2006.01)
*A61B 5/021* (2006.01)

(86) International application number:
**PCT/KR2015/006425**

(87) International publication number:
**WO 2016/017930 (04.02.2016 Gazette 2016/05)**

(54) **HEMADYNAMOMETER AND MOBILE TERMINAL INCLUDING THE SAME**

HÄMODYNAMOMETER UND MOBILES ENDGERÄT DAMIT

HÉMODYNAMOMÈTRE ET TERMINAL MOBILE COMPRENANT CELUI-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.07.2014 KR 20140096368**

(43) Date of publication of application:
**21.06.2017 Bulletin 2017/25**

(73) Proprietor: **LG Electronics Inc.
Seoul 150-721 (KR)**

(72) Inventors:
• **JANG, Seungjin
Seoul 137-893 (KR)**
• **HONG, Seungbum
Seoul 137-893 (KR)**

(74) Representative: **Frenkel, Matthias Alexander
Wuesthoff & Wuesthoff
Patentanwälte PartG mbB
Schweigerstrasse 2
81541 München (DE)**

(56) References cited:
**WO-A1-01/95796          DE-A1-102004 011 681
JP-A- H10 328 151        JP-A- 2003 004 567
KR-A- 20130 125 567    US-A1- 2006 195 035
US-A1- 2010 210 956**

• **WESTERHOF N ET AL: "FORWARD AND
BACKWARD WAVES IN THE ARTERIAL
SYSTEM", CARDIOVASCULAR RESEARCH,
OXFORD UNIVERSITY PRESS, GB, vol. 6, 1
January 1972 (1972-01-01), pages 648-656,
XP001035227, ISSN: 0008-6363, DOI:
10.1093/CVR/6.6.648**
• **ALBERTO P AVOLIO ET AL: "Arterial blood
pressure measurement and pulse wave
analysis--their role in enhancing cardiovascular
assessment", PHYSIOLOGICAL
MEASUREMENT., vol. 31, no. 1, 26 November
2009 (2009-11-26), pages R1-R47, XP055437209,
GB ISSN: 0967-3334, DOI:
10.1088/0967-3334/31/1/R01**
• **ADRIANA ARZA ET AL: "Pulse transit time and
pulse width as potential measure for estimating
beat-to-beat systolic and diastolic blood
pressure", THE INSTITUTE OF ELECTRICAL AND
ELECTRONICS ENGINEERS, INC. (IEEE)
CONFERENCE PROCEEDINGS, 1 September
2013 (2013-09-01), page 887, XP055431131,
Piscataway**

EP 3 179 904 B1

## Description

[Technical Field]

The present invention relates to a hemadynamometer and a mobile terminal including the same, and more particularly to a hemadynamometer capable of simply and accurately measuring a blood pressure, and a mobile terminal including the same.

[Background Art]

In modern society, the percentage of patients suffering from vascular diseases is increasing due to an increase in the percentage of the elderly and dietary habits.

In this regard, research into various schemes for measurement of a blood pressure is being conducted. The blood pressure measurement schemes may include an invasive scheme and a non-invasive scheme. Since the invasive scheme entails user inconvenience, the non-invasive scheme, which is based on a pressure, has recently commanded attention.

DE 10 2004 011 681 A1 discloses a blood pressure monitor which comprises a first transmitter/receiver pair, a second transmitter/receiver pair and a pressurization unit for changing a radial dimension of a blood vessel. The first and second transmitter/receiver pairs are configured to detect a pulse signal at locations of a body where each of the first and second transmitter/receiver pairs is disposed. The pulse signals are observed over time while changing the pressure exerted by the pressurization unit on the blood vessel. A systolic pressure is determined by a sudden rise of the pulse signal detected by the second/transmitter receiver pair and a maximum pulse signal value detected by the first transmitter/receiver pair while the pressure exerted by the pressurization unit decreases. It is also disclosed to determine a phase difference over time between the pulse signal detected by the first transmitter/receiver pair and the second transmitter/receiver pair in dependence on the pressure exerted by the pressurization unit, and to determine the systolic pressure and the diastolic pressure based on the determined phase difference over time.

However, the pressure-based non-invasive scheme has an issue in that it is difficult to accurately measure a blood pressure and an associated device is inconvenient to carry.

[Disclosure]

[Technical Problem]

Therefore, the present invention has been made in view of the above problems, and it is an object of the present invention to provide a hemadynamometer capable of simply and accurately measuring a blood pressure, and a mobile terminal including the same.

[Technical Solution]

The present invention is defined by the subject-matter of independent claim 1. In accordance with an aspect of the present invention, the above and other objects can be accomplished by the provision of a hemadynamometer including a first pulse wave sensor and a second pulse wave sensor disposed apart from each other, each of the first pulse wave sensor and the second pulse wave sensor converting a pulse wave signal corresponding to a blood pressure into an electric signal, a pressurization unit disposed between the first pulse wave sensor and the second pulse wave sensor to apply a pressure to a wrist to change a diameter of a blood vessel, and a controller to measure the blood pressure based on first and second pulse wave signals detected respectively by the first pulse wave sensor and the second pulse wave sensor before a predetermined pressure is applied to the wrist by the pressurization unit, and third and fourth pulse wave signals detected respectively by the first pulse wave sensor and the second pulse wave sensor after the predetermined pressure is applied to the wrist by the pressurization unit.

In accordance with another aspect of the present invention, there is provided a mobile terminal including a communication unit, a display to display information received from the communication unit, a blood pressure measurement unit including a first pulse wave sensor and a second pulse wave sensor disposed apart from each other, each of the first pulse wave sensor and the second pulse wave sensor converting a pulse wave signal corresponding to a blood pressure into an electric signal, and a pressurization unit disposed between the first pulse wave sensor and the second pulse wave sensor to apply a pressure to a wrist to change a diameter of a blood vessel, and a controller to measure the blood pressure based on first and second pulse wave signals detected respectively by the first pulse wave sensor and the second pulse wave sensor before a predetermined pressure is applied to the wrist by the pressurization unit, and third and fourth pulse wave signals detected respectively by the first pulse wave sensor and the second pulse wave sensor after the predetermined pressure is applied to the wrist by the pressurization unit.

## Brief Description of Drawings

The above and other objects, features and other advantages of the present invention will be more clearly understood from the following detailed description taken in conjunction with the accompanying drawings, in which:

FIG. 1 is a view illustrating an example of a hemadynamometer according to an embodiment of the present invention;
FIG. 2 is a view referred to for comparison with the hemadynamometer of FIG. 1;

FIGS. 3A to 3C are views referred to for describing various blood vessels and blood flow velocities in the blood vessels;

FIGS. 4A to 4C are views referred to for describing a principle of superposition of pulse waves;

FIG. 5 is a block diagram schematically showing an internal configuration of the hemadynamometer of FIG. 1;

FIG. 6A is a view illustrating a shape of a blood vessel before a predetermined pressure is applied to the hemadynamometer of FIG. 1;

FIG. 6B is a view illustrating a shape of the blood vessel after the predetermined pressure is applied to the hemadynamometer of FIG. 1;

FIG. 6C is a waveform diagram of pulse wave signals detected by first and second pulse wave sensors in the shapes of FIGS. 6A and 6B;

FIGS. 7A to 7D are views illustrating various audio outputs of the hemadynamometer of FIG. 1;

FIG. 8 is a view illustrating an example of a hemadynamometer according to another embodiment of the present invention;

FIG. 9A is a view illustrating that a mobile terminal equipped with the hemadynamometer of FIG. 1 is worn on a wrist;

FIG. 9B is a perspective view of the wearable mobile terminal of FIG. 9A; and

FIG. 10 is a block diagram showing an internal configuration of the wearable mobile terminal of FIG. 9A or 9B.

## Best Mode for Carrying out the Invention

Exemplary embodiments of the present invention will be described with reference to the attached drawings.

The terms "module" and "unit" used in description of components are used herein to aid in understanding of the components and thus should not be misconstrued as having specific meanings or roles. Accordingly, the terms "module" and "unit" may be used interchangeably.

A hemadynamometer and a mobile terminal equipped with the hemadynamometer described in this specification are a hemadynamometer simply attachable to a case and available for medication counseling, and a mobile terminal equipped with the hemadynamometer. Hereinafter, the hemadynamometer and the mobile terminal equipped with the hemadynamometer will be described in detail.

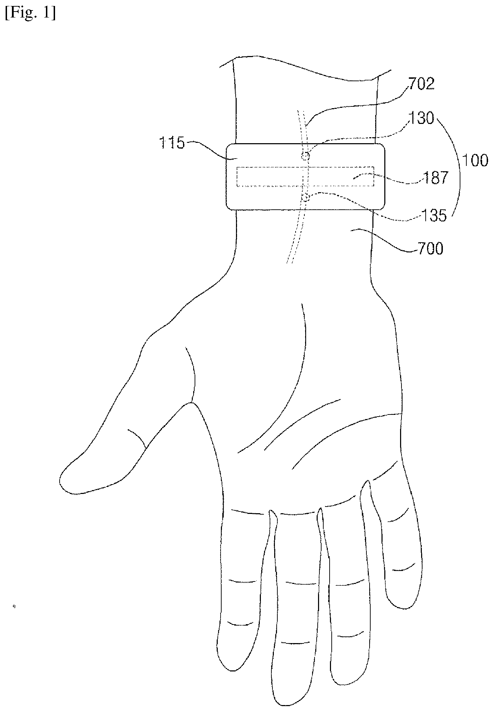

FIG. 1 illustrates an example of a hemadynamometer according to an embodiment of the present invention.

Referring to FIG. 1, the hemadynamometer according to the present embodiment, denoted by reference numeral 100, includes a first pulse wave sensor 130 and a second pulse wave sensor 135 disposed apart from each other. Each of the first pulse wave sensor 130 and the second pulse wave sensor 135 converts a pulse wave signal corresponding to a blood pressure into an electric signal. The hemadynamometer 100 further includes a pressurization unit 187 disposed between the first pulse wave sensor 130 and the second pulse wave sensor 135 for applying a pressure to a wrist 700 to change a diameter of a blood vessel.

The hemadynamometer 100 measures the blood pressure based on first and second pulse wave signals detected respectively by the first pulse wave sensor 130 and the second pulse wave sensor 135 before a predetermined pressure is applied to the wrist 700 by the pressurization unit 187, and third and fourth pulse wave signals detected respectively by the first pulse wave sensor 130 and the second pulse wave sensor 135 after the predetermined pressure is applied to the wrist 700 by the pressurization unit 187.

The first pulse wave sensor 130, the second pulse wave sensor 135 and the pressurization unit 187 in the hemadynamometer 100 are attached to a band 115 wearable on the wrist 700.

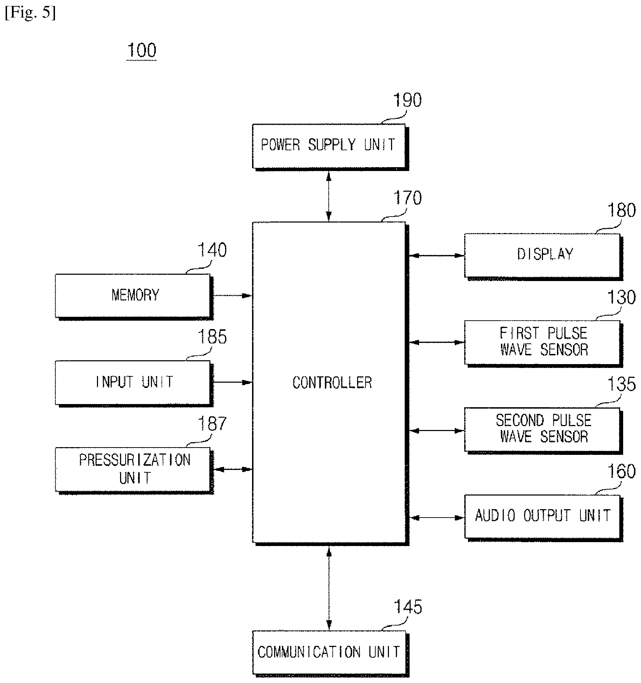

To measure a blood pressure at a radial artery 702, the hemadynamometer 100 may be worn such that the first pulse wave sensor 130, the second pulse wave sensor 135 and the pressurization unit 187 are disposed near the radial artery 702. Each of the first pulse wave sensor 130 and the second pulse wave sensor 135 in the hemadynamometer 100 may convert a pulse wave signal detected on the radial artery 702 in the wrist 700 into an electric signal, which may then be transmitted to a controller (denoted by reference numeral 170 in FIG. 5) included in the hemadynamometer 100.

The hemadynamometer 100 separates first to fourth forward wave signals and first to fourth backward wave signals respectively from the first to fourth pulse wave signals, and measure a blood pressure based on at least one of a difference in pulse width or a difference in peak value between the first and third backward wave signals and a difference in pulse width or a difference in peak value between the second and fourth forward wave signals.

The hemadynamometer 100 may measure the blood pressure in proportion to the difference in pulse width or the difference in peak value.

The hemadynamometer 100 may output a message for adjustment of positions of the first pulse wave sensor 130, the second pulse wave sensor 135 and the pressurization unit 187, or a remeasurement notification message when the difference in pulse width is less than or equal to a first predetermined value, the difference in peak value is less than or equal to a second predetermined value, or the first to fourth pulse wave signals have levels less than or equal to a third predetermined value.

The hemadynamometer 100 may measure the blood pressure based on the first and second pulse wave signals detected respectively by the first pulse wave sensor 130 and the second pulse wave sensor 135 in a state in which a first pressure is transmitted to the wrist 700 by the pressurization unit 187, and the third and fourth pulse wave signals detected respectively by the first pulse wave sensor 130 and the second pulse wave sensor 135 in a state in which a second pressure is transmitted to the wrist 700 by the pressurization unit 187.

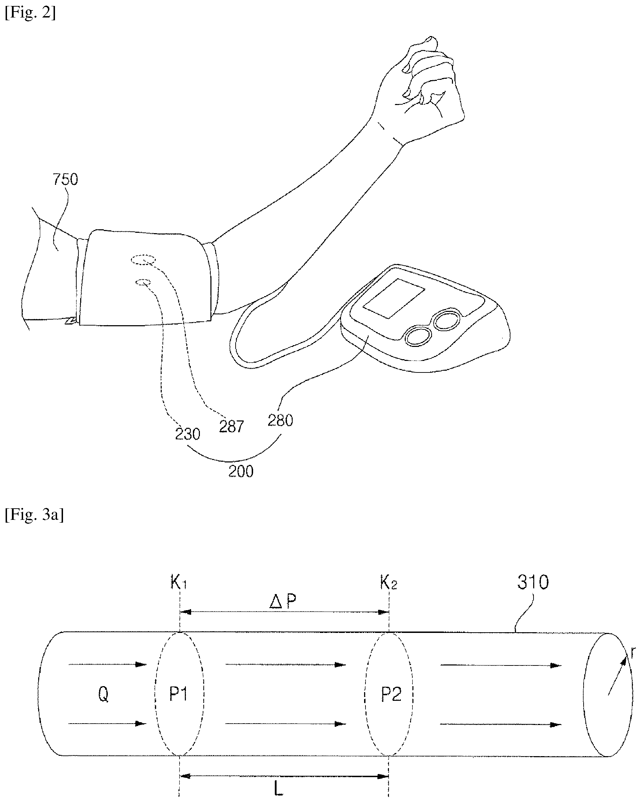

FIG. 2 is referred to for comparison with the hemadynamometer 100 of FIG. 1.

A hemadynamometer 200 of FIG. 2 is a general hemadynamometer provided in a hospital or the like, and includes a pulse wave sensor 230 and a pressurization unit 287 attached to an arm of a user.

The pulse wave sensor 230 and the pressurization unit 287 are used in the following manner. That is, the pressurization unit 287 is operated to apply a maximum pressure, and then the pulse wave sensor 230 is operated to measure pulse waves at the same position while the pressure is gradually lowered.

A blood pressure is measured using a variation in pulse wave at a particular position with a variation in pressure.

The use of the hemadynamometer 200 does not consider a variation in ambient temperature or body temperature and requires time to change a pressure, resulting in user inconvenience. In particular, the hemadynamometer 200 is difficult to carry.

In order to solve the above-described issues, the present invention proposes the hemadynamometer 100 as in FIG. 1.

The hemadynamometer 100 according to the present embodiment may easily and accurately measure a blood pressure by including the first pulse wave sensor 130 and the second pulse wave sensor 135 which are disposed apart from each other, and each convert a pulse wave signal corresponding to the blood pressure into an electric signal, and the pressurization unit 187 which is disposed between the first pulse wave sensor 130 and the second pulse wave sensor 135 to apply a pressure to the wrist 700 to change a diameter of a blood vessel.

In particular, it is possible to estimate a diameter of a blood vessel by detecting pulse waves at two or more positions in the blood vessel while changing the diameter of the blood vessel using the pressurization unit 187, and it is possible to measure the blood pressure based on the estimated diameter.

Therefore, it is possible to easily and accurately measure the blood pressure reflecting an ambient temperature change or a body temperature change without a separate device.

In addition, the first and second pulse wave sensors 130 and 135, the pressurization unit 187, and the like are attached to the band 115, so that the hemadynamometer 100 may be conveniently worn on the wrist 700 or the like.

Hereinafter, a description will be given of a basic principle of a scheme of measuring the blood pressure described in the present invention with reference to FIGS. 3A to 4C.

FIGS. 3A to 3C are referred to for describing various blood vessels and blood flow velocities in the blood vessels.

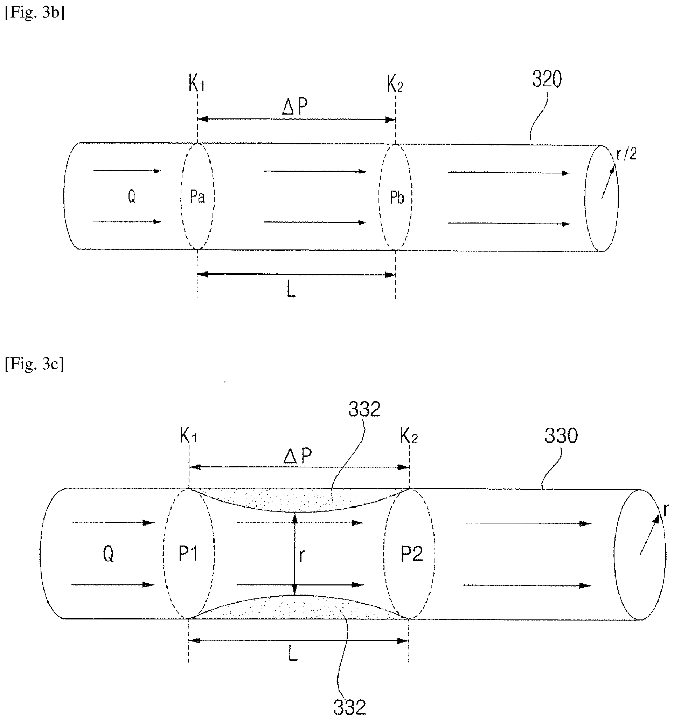

FIG. 3A illustrates a blood vessel 310 having a radius r, FIG. 3B illustrates a blood vessel 320 having a radius r/2, and FIG. 3C illustrates a blood vessel 330 having a radius r and including blood clots 332.

FIG. 3A is referred to for describing the blood vessel 310 and a blood flow velocity in the blood vessel 310.

In a basic principle of hemodynamics, the blood vessel 310 may be regarded as a pipe having a resistance and an elasticity.

A blood flow F passing through the blood vessel 310 is proportional to a blood pressure P, and is inversely proportional to a blood flow resistance R. That is, the blood flow F may be expressed by an equation F = P/R. When the equation is transformed into an equation P = F x R (blood pressure = blood flow x blood flow resistance), the blood pressure P increases as the blood flow F or the blood flow resistance R increases, and decreases as the blood flow F or the blood flow resistance R decreases.

Poiseuille's law is a significant law in hemodynamics. Poiseuille's law is expressed by the following Equation 1.

[Equation 1]

$$Q = \frac{\Delta P}{R}$$

Here, Q denotes a blood flow velocity, $\Delta P$ denotes a difference in blood pressure between a position K1 and a position K2, and R denotes a blood flow resistance.

According to Poiseuille's law of Equation 1, the blood flow velocity Q is proportional to the difference in blood pressure $\Delta P$ in a unit section (length L), and is inversely proportional to the blood flow resistance R.

The blood flow F or the blood flow velocity Q may be expressed by the following Equation 2.

[Equation 2]

$$F \; or \; Q = \frac{\Delta P \cdot r^4 \cdot \pi}{\eta \cdot L \cdot 8}$$

Here, $\eta$ denotes a viscosity of blood, L denotes a length of the blood vessel 310 between the position K1 and the position K2, $\Delta P$ denotes the difference in blood pressure between the position K1 and the position K2, and r denotes a radius of the blood vessel 310.

Equations 3 and 4 are obtained using Equations 1 and 2.

[Equation 3]

$$Q = \frac{\Delta P}{R} = \frac{\Delta P \cdot r^4 \cdot \pi}{\eta \cdot L \cdot 8}$$

[Equation 4]

$$R = \frac{\eta \cdot L \cdot 8}{r^4 \cdot \pi}$$

According to Equation 4, the blood flow resistance R is proportional to the viscosity $\eta$ and the length L of the blood vessel 310, and is inversely proportional to the radius r of the blood vessel 310 to the power of four.

When Poiseuille's law is applied to a blood vessel system of a common person, a difference in blood pressure $\Delta P$ may be regarded as being constant since a constant arterial blood pressure is maintained for a short period of time. Similarly, the viscosity $\eta$ of blood that varies with a haematocrit and a temperature may be regarded as being almost constant. When the length L of the blood vessel 310 is known and is regarded as being constant, only the radius r of the blood vessel 310 is a variable in the equation related to the blood flow resistance R.

The radius r of the blood vessel 310 is the only factor that changes the blood flow resistance R, and is a significant variable of the blood pressure P.

For example, when an ambient temperature rapidly decreases as a person moves from the inside where it is warn to the outside where it is cold, a blood vessel muscle contracts to maintain a body temperature due to a homeostatic mechanism. As a result, when the blood vessel 320 has a radius decreased to 1/2 of the radius r as shown in FIG. 3B, the blood flow F decreases to 1/16 thereof corresponding to 1/2 to the power of four on the assumption that the blood pressure P is constant.

Then, it is difficult to supply a body with oxygen and nutrients as needed and thus, the blood pressure P inevitably increases to supply a sufficient blood flow. As a result of this principle, brain hemorrhage and cerebral aneurysm frequently occur in winter.

Meanwhile, referring to FIG. 3C, when the blood clots 332 are generated in the blood vessel 330, and a portion corresponding to the blood clots 332 has a diameter r, that is, the blood vessel 330 substantially has a radius r/2, the blood flow F decreases to 1/16 thereof corresponding to 1/2 to the power of four on the assumption that the blood pressure P is constant as shown in FIG. 3B. Then, it is difficult to supply a body with oxygen and nutrients as needed and thus, the blood pressure P inevitably increases to supply a sufficient blood flow.

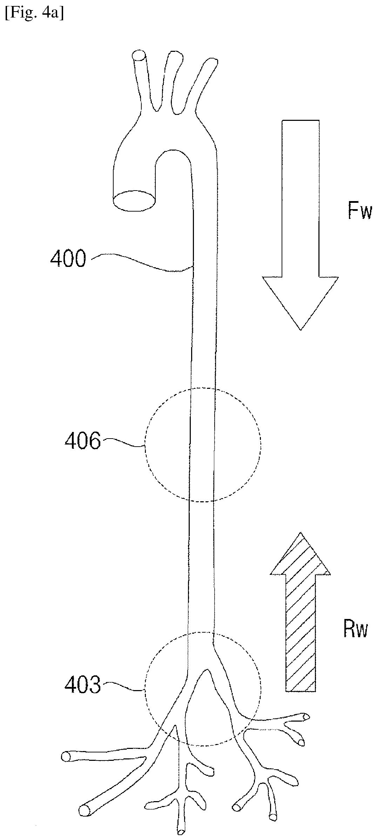

FIG. 4A to FIG. 4C are referred to for describing a principle of superposition of pulse waves.

FIG. 4A illustrates a direction of a blood flow in a blood vessel 400.

The blood vessel 400 has a diameter which is the thickest and largest at the origin of the aorta, and is continuously decreasing toward peripheral blood vessels. In this instance, a backward wave or reflected wave signal and a forward wave signal of a peripheral blood vessel portion generated due to a blood flow resistance R are simultaneously observed.

A pulse wave observed at an arbitrary middle point 406 is a mixed wave, and has a waveform in which a forward wave signal FW overlaps a backward wave signal RW.

The arbitrary middle point 406 may correspond to a radial artery portion of the wrist 700.

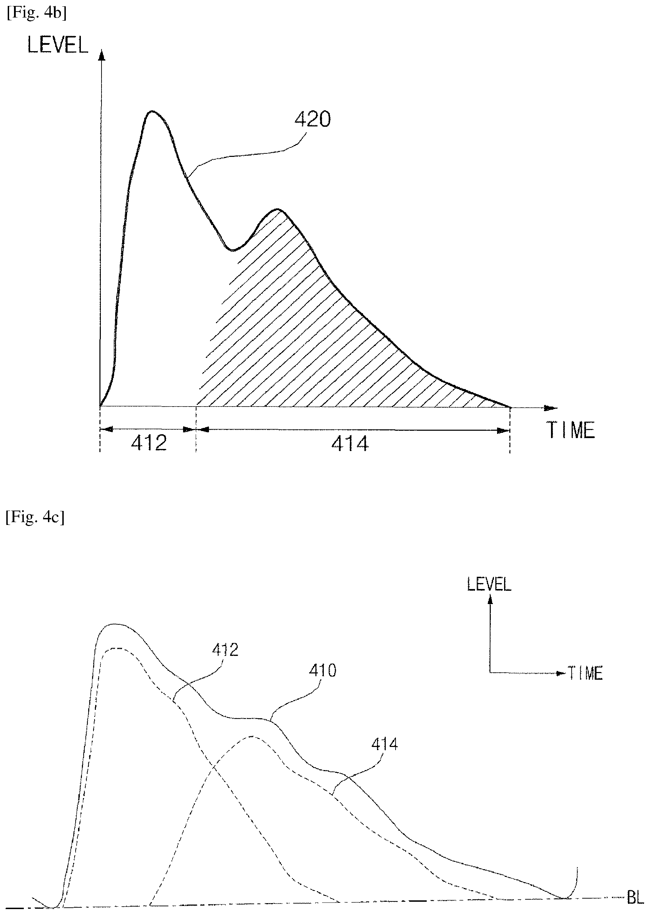

FIG. 4B illustrates that a forward wave signal FW 412 and a backward wave signal RW 414 generated at the middle point 406 of FIG. 4A are conceptually separated from each other.

In practice, the signals are indicated as a mixed wave (cuff pulse wave) 410 as illustrated in FIG. 4C.

In the present invention, signal processing is performed on the mixed wave 410 to separate the mixed wave 410 into the forward wave signal 412 and the backward wave signal 414, and a blood pressure is measured using a difference in pulse width or peak value and the like between the forward wave signal 412 and the backward wave signal 414. In this instance, the separation into the forward wave signal 412 and the backward wave signal 414 may be performed based on at least one of a variation in slope and a variation in amplitude of the mixed wave. The separation into the forward wave signal 412 and the backward wave signal 414 may be performed by the controller 170.

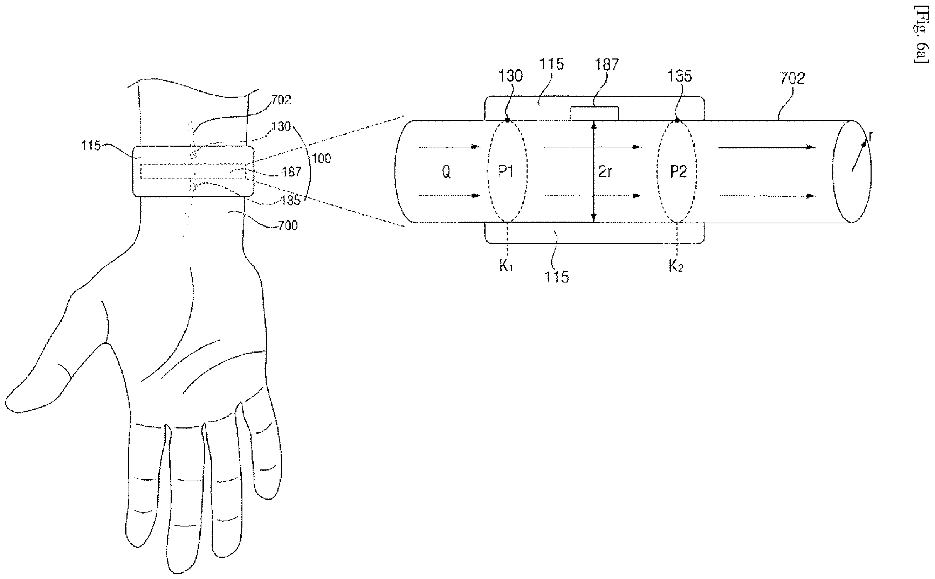

A scheme of measuring the blood pressure using the hemadynamometer 100 according to the present embodiment will be described in detail with reference to FIG. 6A and the following figures.

FIG. 5 schematically shows an internal configuration of the hemadynamometer 100 of FIG. 1.

Referring to FIG. 5, the hemadynamometer 100 may include the first pulse wave sensor 130, the second pulse wave sensor 135, a communication unit 145, a memory 140, an audio output unit 160, the controller 170, a display 180, the pressurization unit 187, and a power supply unit 190. When the components are implemented in a practical application, two or more components may be combined into one component, or a component may be divided into two or more components.

The first pulse wave sensor 130 and the second pulse wave sensor 135 are disposed apart from each other. In particular, the sensors are preferably disposed opposite each other around the pressurization unit 187.

The first pulse wave sensor 130 and the second pulse wave sensor 135 may be attached to the band 115 together with the pressurization unit 187. In particular, the first pulse wave sensor 130, the second pulse wave sensor 135 and the pressurization unit 187 are preferably disposed near the radial artery 702 of the wrist 700 of FIG. 1.

The first pulse wave sensor 130 and the second pulse wave sensor 135 detect pulse wave signals at a first position and a second position, respectively, of the radial artery 702. In particular, each of the sensors converts a pulse wave signal corresponding to a blood pressure into an electric signal.

Each of the pulse wave signals detected by the first pulse wave sensor 130 and the second pulse wave sensor 135 is transmitted to the controller 170.

In particular, the first pulse wave sensor 130 and the second pulse wave sensor 135 transmits the first and second pulse wave signals, respectively, to the controller 170. Here, the first and second pulse wave signals are detected before a predetermined pressure is applied to the wrist 700 by the pressurization unit 187. In addition, the first pulse wave sensor 130 and the second pulse wave sensor 135 transmits the third and fourth pulse wave signals, respectively, to the controller 170. Here, the third and fourth pulse wave signals are detected after the predetermined pressure is applied to the wrist 700 by the pressurization unit 187.

Each of the first to fourth pulse wave signals is divided into a forward wave signal and a backward wave signal as described with reference to FIGS. 4A to 4C. The signals is separated by the controller 170.

The communication unit 145 may provide an interface for communication with an external device. To this end, the communication unit 145 may include at least one of a mobile communication module (not illustrated), a wireless Internet module (not illustrated), a near field communication module (not illustrated), a global positioning system (GPS) module (not illustrated), and the like.

For example, the communication unit 145 may perform Bluetooth communication, wireless fidelity (WiFi) communication, or the like, thereby transmitting information about a blood pressure measured by the controller 170 to a paired mobile terminal. In this instance, the mobile terminal may be a smart phone or the like capable of performing voice communication.

The memory 140 may store a program for processing or control of the controller 170 in the hemadynamometer 100, and perform a function of temporarily storing input or output data.

In particular, the memory 140 may store the information about the blood pressure measured by the controller 170 in the hemadynamometer 100 together with time information. Furthermore, the memory 140 may store user information.

The audio output unit 160 may output the information about the measured blood pressure as an audio. The audio output unit 160 may output a message for adjustment of positions of the first pulse wave sensor 130, the second pulse wave sensor 135 and the pressurization unit 187 as an audio, or output a remeasurement notification message as an audio.

The audio output unit 160 may output information necessary for an operation of the hemadynamometer as an audio.

The pressurization unit 187 is disposed between the first pulse wave sensor 130 and the second pulse wave sensor 135. The pressurization unit 187 applies a pressure to the wrist 700 to change a diameter of a blood vessel.

When an input unit 185 receives an input for the measurement of the blood pressure, the pressurization unit 187 may be expanded. For example, when the pressurization unit 187 is an inflatable unit, the pressurization unit 187 is expanded using a pump, thereby decreasing the diameter of the blood vessel.

Each of the first pulse wave sensor 130 and the second pulse wave sensor 135 detects a pulse wave signal according to a variation in diameter of the blood vessel, and the controller 170 may calculate the blood pressure based on the pulse wave signal according to the variation in diameter of the blood vessel.

The controller 170 may control operations of the respective units in the hemadynamometer 100 to control an overall operation of the hemadynamometer 100.

For example, the controller 170 measures the blood pressure based on the first and second pulse wave signals detected respectively by the first pulse wave sensor 130 and the second pulse wave sensor 135 before a predetermined pressure is applied to the wrist 700 by the pressurization unit 187, and the third and fourth pulse wave signals detected respectively by the first pulse wave sensor 130 and the second pulse wave sensor 135 after the predetermined pressure is applied to the wrist 700 by the pressurization unit 187.

The controller 170 separates the first to fourth forward wave signals and the first to fourth backward wave signals respectively from the first to fourth pulse wave signals based on at least one of a variation in slope and a variation in amplitude of each of the pulse wave signals, and measure the blood pressure based on at least one of a difference in pulse width or a difference in peak value between the first and third backward wave signals and a difference in pulse width or a difference in peak value between the second and fourth forward wave signals.

The controller 170 may measure the blood pressure in proportion to the difference in pulse width or the difference in peak value.

When the difference in pulse width is less than or equal to the first predetermined value, the difference in peak value is less than or equal to the second predetermined value, or the first to fourth pulse wave signals have levels less than or equal to the third predetermined value, the controller 170 may control a display 180 or the audio output unit 160 to output the message for adjustment of positions of the first pulse wave sensor 130, the second pulse wave sensor 135 and the pressurization unit 187, or the remeasurement notification message.

The controller 170 may measure the blood pressure based on the first and second pulse wave signals detected respectively by the first pulse wave sensor 130 and the second pulse wave sensor 135 in a state in which the first pressure is transmitted to the wrist 700 by the pressurization unit 187, and the third and fourth pulse wave signals detected respectively by the first pulse wave sensor 130 and the second pulse wave sensor 135 in a state in which the second pressure is transmitted to the wrist 700 by the pressurization unit 187.

The display 180 may display text, an image, or the like.

In particular, the display 180 may output the information about the blood pressure.

The display 180 may output the message for adjustment of positions of the first pulse wave sensor 130, the second pulse wave sensor 135 and the pressurization unit 187, or the remeasurement notification message.

The input unit 185 may include a button and the like to allow an input for the measurement of the blood pressure.

The power supply unit 190 may supply power necessary for an operation of each component under control of the controller 170.

FIG. 6A illustrates a shape of the blood vessel before a predetermined pressure is applied to the hemadynamometer 100 of FIG. 1.

Referring to FIG. 6A, the pressurization unit 187 in the hemadynamometer 100 is positioned near the radial artery 702, and the predetermined pressure is to be applied. Therefore, the blood vessel has a diameter 2r at the pressurization unit 187.

In this instance, each of the first pulse wave sensor 130 and the second pulse wave sensor 135 may measure a pulse wave signal.

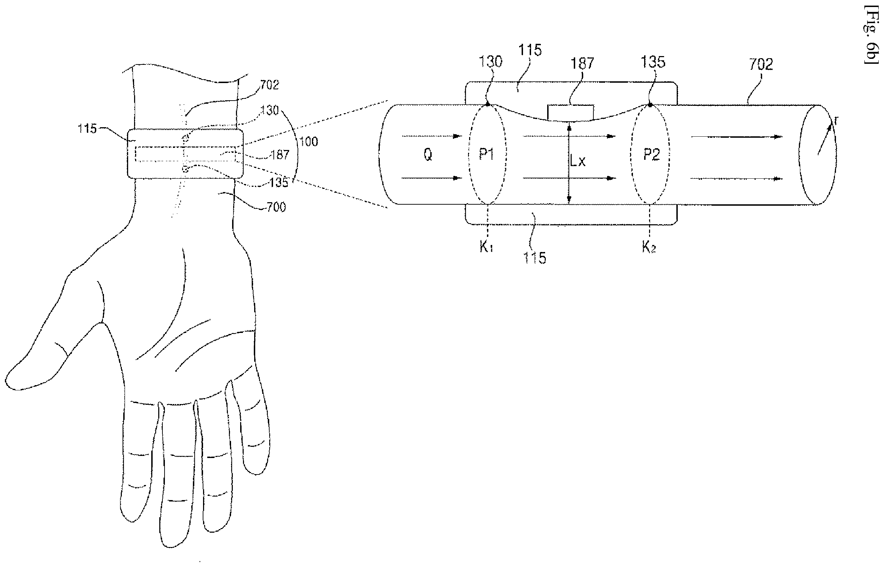

FIG. 6B illustrates a shape of the blood vessel after the predetermined pressure is applied to the hemadynamometer 100 of FIG. 1.

Referring to FIG. 6B, the pressurization unit 187 in the hemadynamometer 100 is positioned near the radial artery 702, and the predetermined pressure is applied. Therefore, the blood vessel at the pressurization unit 187 may have a diameter Lx, which is less than 2r.

In this instance, each of the first pulse wave sensor 130 and the second pulse wave sensor 135 may measure a pulse wave signal.

When it is presumed that the blood flow is constant without an environmental change based on Poiseuille's law, pulse waves similar to each other are observed at positions of the first pulse wave sensor 130 and the second pulse wave sensor 135 before the pressure is applied as illustrated in FIG. 6A.

When the pressure is applied as illustrated in FIG. 6B, the diameter of the blood vessel decreases to Lx due to the pressure, and the radius of the blood vessel decreases. Thus, a difference in blood pressure $\Delta P$ increases to send a constant blood flow.

Therefore, at the first position K1 where the first pulse wave sensor 130 is positioned, the blood flow resistance R increases due to a decreased blood vessel diameter at the pressurization unit 187 and thus a backward wave has an increased peak value and an increased pulse width.

However, at the second position K2 where the second pulse wave sensor 135 is positioned, the blood flow velocity temporarily increases near a bottleneck due to Beroulli's law. Thus, a forward wave has an increased peak value and a decreased pulse width.

In the present invention, a relative change of a blood vessel diameter is deduced based on a variation in pulse width and amplitude of each of a forward wave and a backward wave in response to a pressure applied, and the blood pressure may be deduced using a form and velocity analysis (pulse transition time (PTT)) of a pulse wave by deducing the change of the diameter.

The controller 170 may calculate a blood vessel diameter that decreases as a pulse width of a backward wave at the first position K1 where the first pulse wave sensor 130 is positioned increases after a pressure is applied, and the controller 170 may calculate a blood vessel diameter that decreases as amplitude of a forward wave at the second position K2 where the second pulse wave sensor 135 is positioned increases after the pressure is applied.

Accordingly, the controller 170 may calculate the blood pressure that increases when a blood vessel diameter decreases as a pulse width of a backward wave at the first position K1 where the first pulse wave sensor 130 is positioned increases after a pressure is applied, and the controller 170 may calculate the blood pressure that increases when a blood vessel diameter decreases as an amplitude of a forward wave at the second position K2 where the second pulse wave sensor 135 is positioned increases after the pressure is applied.

This is a measurement scheme distinct from the hemadynamometer 200 of FIG. 2 using a pulse wave sensor, and allows accurate and simple measurement of the blood pressure. In addition, the hemadynamometer 100 may be worn on the wrist 700 or the like, and thus is portable. Thus, user convenience is enhanced.

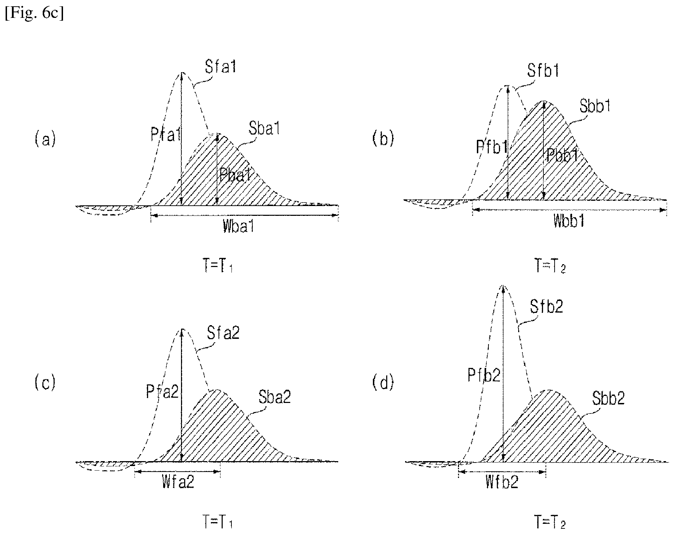

FIG. 6C illustrates pulse wave signals detected by first and second pulse wave sensors in the shapes of FIGS. 6A and 6B.

Referring to FIG. 6C, FIG. 6C(a) depicts a first forward wave signal Sfa1 and a first backward wave signal Sba1 extracted from the first pulse wave signal which is detected by the first pulse wave sensor 130 before a pressure is applied at T1.

FIG. 6C(b) depicts a third forward wave signal Sfb1 and a third backward wave signal Sbb1 extracted from the third pulse wave signal which is detected by the first pulse wave sensor 130 after the pressure is applied at T2.

The controller 170 may measure a pressure based on a difference in pulse width or a difference in peak value between the first and third backward wave signals.

When FIG. 6C(a) is compared with FIG. 6C(b), a peak value Pfa1 of the first forward wave signal Sfa1 and a peak value Pfb1 of the third forward wave signal Sfb1 are not significantly different from each other and thus are not used when a blood pressure is measured.

When FIG. 6C(a) is compared with FIG. 6C(b), a peak value Pba1 of the first backward wave signal Sba1 and a peak value Pbb1 of the third backward wave signal Sbb1 are significantly different from each other and thus are used when a blood pressure is measured. It is possible to measure a blood pressure in proportion to a difference between the peak value Pba1 of the first backward wave signal Sba1 and the peak value Pbb1 of the third backward wave signal Sbb1.

When FIG. 6C(a) is compared with FIG. 6C(b), a pulse width Wba1 of the first backward wave signal Sba1 and a pulse width Wbb1 of the third backward wave signal Sbb1 are significantly different from each other and thus are used when a blood pressure is measured. It is possible to measure a blood pressure in proportion to a difference between the pulse width Wba1 of the first backward wave signal Sba1 and the pulse width Wbb1 of the third backward wave signal Sbb1.

It is possible to measure a blood pressure based on both the difference between the peak value Pba1 of the first backward wave signal Sba1 and the peak value Pbb1 of the third backward wave signal Sbb1 and the difference between the pulse width Wba1 of the first backward wave signal Sba1 and the pulse width Wbb1 of the third backward wave signal Sbb1.

FIG. 6C(c) depicts a second forward wave signal Sfa2 and a second backward wave signal Sba2 extracted from the second pulse wave signal which is detected by the second pulse wave sensor 135 before the pressure is applied at T1.

FIG. 6C(d) depicts a fourth forward wave signal Sfb2 and a fourth backward wave signal Sbb2 extracted from the second pulse wave signal which is detected by the second pulse wave sensor 135 after the pressure is applied at T2.

The controller 170 may measure a pressure based on a difference in pulse width or a difference in peak value between the second and fourth forward wave signals.

When FIG. 6C(c) is compared with FIG. 6C(d), a peak value or a pulse width of the second backward wave signal Sba2 and a peak value or a pulse width of the fourth backward wave signal Sbb2 are not significantly different from each other and thus are not used when a blood pressure is measured.

When FIG. 6C(c) is compared with FIG. 6C(d), a peak value Pfa2 of the second forward wave signal Sfa2 and a peak value Pfb2 of the fourth forward wave signal Sfb2 are significantly different from each other and thus are used when a blood pressure is measured. It is possible to measure a blood pressure in proportion to a difference between the peak value Pfa2 of the second forward wave signal Sfa2 and the peak value Pfb2 of the fourth forward wave signal Sfb2.

When FIG. 6C(c) is compared with FIG. 6C(d), a pulse width Wfa2 of the second forward wave signal Sfa2 and a pulse width Wfb2 of the fourth forward wave signal Sfb2 are significantly different from each other and thus are used when a blood pressure is measured. It is possible to measure a blood pressure in proportion to a difference between the pulse width Wfa2 of the second forward wave signal Sfa2 and the pulse width Wfb2 of the fourth forward wave signal Sfb2.

It is possible to measure a blood pressure based on both the difference between the pulse width Wfa2 of the second forward wave signal Sfa2 and the pulse width Wfb2 of the fourth forward wave signal Sfb2 and the difference between the peak value Pfa2 of the second forward wave signal Sfa2 and the peak value Pfb2 of the fourth forward wave signal Sfb2.

It is possible to detect the first and second pulse wave signals by the first pulse wave sensor 130 and the second pulse wave sensor 135, respectively, after a first pressure is applied, detect the third and fourth pulse wave signals by the first pulse wave sensor 130 and the second pulse wave sensor 135, respectively, after a second pressure is applied, and measure a blood pressure based on at least one of pulse widths and peak values of the first to fourth pulse wave signals.

According to a scheme of analyzing a pulse wave signal of FIG. 6C, the controller 170 may estimate or calculate a thickness of a blood vessel according to Equations 1 to 4 based on a variation in pulse width or a variation in peak value of a forward wave signal or a backward wave signal, and measure or calculate a blood pressure based on the thickness.

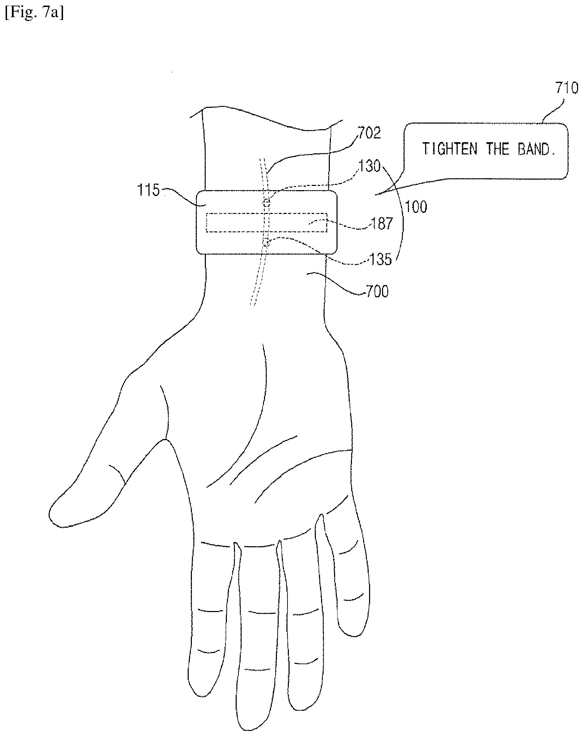

FIGS. 7A to 7D illustrate various audios of the hemadynamometer 100 of FIG. 1.

FIG. 7A illustrates that an audio 710 saying "Tighten the band" is output from the audio output unit 160 of the hemadynamometer 100.

For example, when an input for measurement of the blood pressure is received in response to a user operating the input unit 185, the controller 170 may perform a control operation to output the audio 710 saying "Tighten the band".

The audio 710 may be output when a user wears the band.

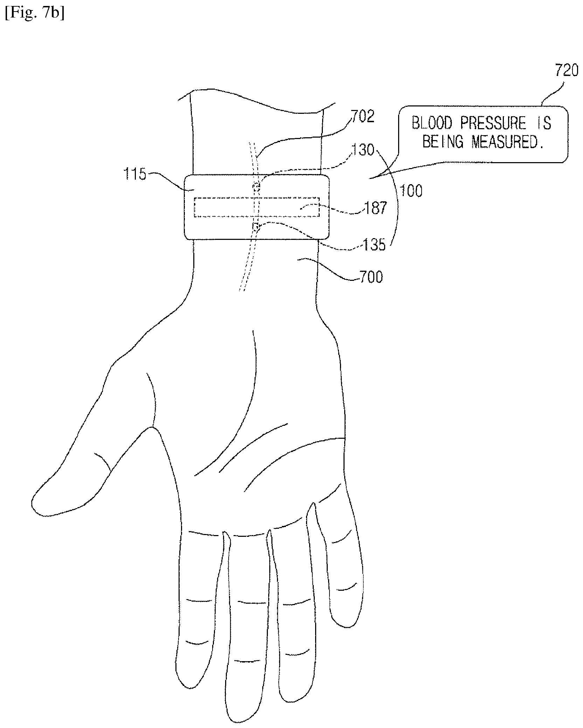

FIG. 7B illustrates that an audio 720 saying "Blood pressure is being measured" is output from the audio output unit 160 of the hemadynamometer 100.

For example, the controller 170 may perform a control operation to output the audio 720 saying "Blood pressure is being measured" when pulse wave signals are detected by the first pulse wave sensor 130 and the second pulse wave sensor 135 while or after a pressure is applied to an area in which the pressurization unit 187 is located in response to the pressurization unit 187 operating. The audio may allow a user to recognize that the blood pressure is being measured. In this instance, it is preferable that the user minimize motion.

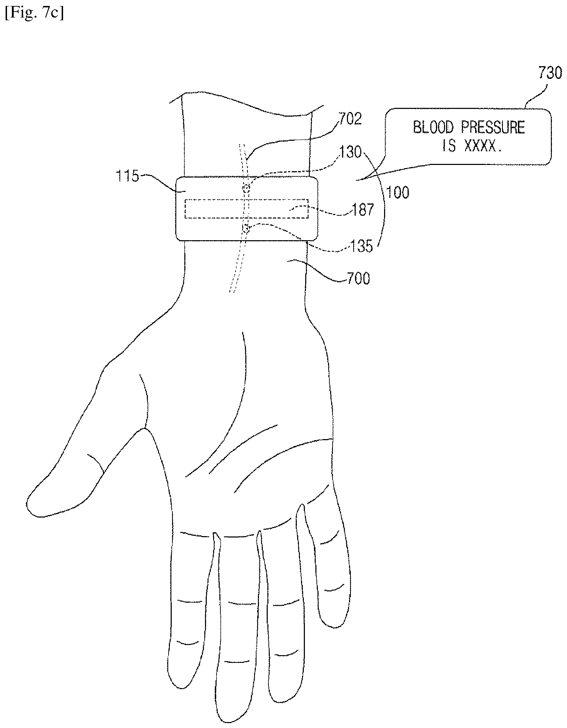

FIG. 7C illustrates that an audio 730 saying "Blood pressure is XXXX" is output from the audio output unit 160 of the hemadynamometer 100.

When blood pressure measurement is completed, the controller 170 may perform a control operation to output blood pressure information as an audio or a video through the audio output unit 160 or the display 180.

When the blood pressure measurement is completed, the controller 170 may transmit the blood pressure information to a mobile terminal 600 paired therewith. Thus, the mobile terminal 600 may manage the measured blood pressure information of a user.

For example, a health related application for a user installed in the mobile terminal 600 may store and manage information about frequently measured blood pressures, and display the blood pressure information using a graphical user interface for a predetermined period of time.

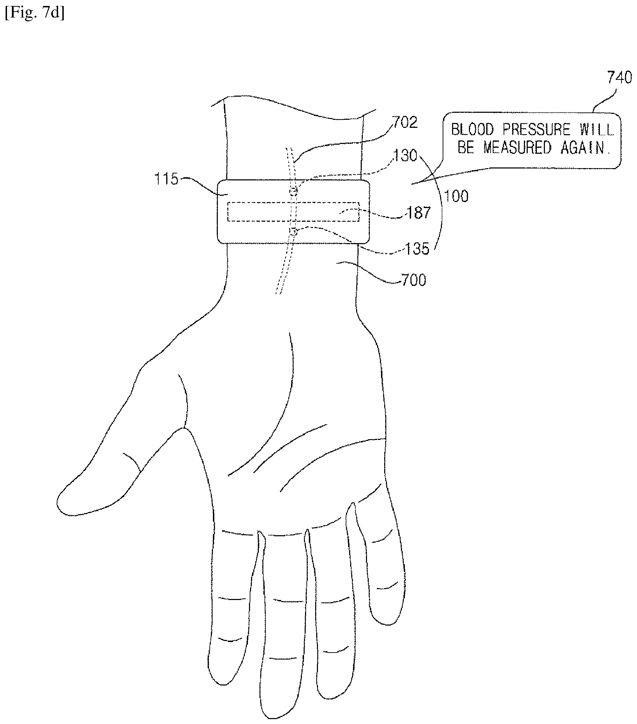

FIG. 7D illustrates that an audio 740 saying "Blood pressure will be measured again" is output from the audio output unit 160 of the hemadynamometer 100.

For example, the controller 170 may perform a control operation to output the audio 740 saying "Blood pressure will be measured again" when a difference in pulse width between the first and third backward wave signals or a difference in pulse width between the second and fourth forward wave signals is less than or equal to a first predetermined value, a difference in peak value between the first and third backward wave signals or a difference in peak value between the second and fourth forward wave signals is less than or equal to a second predetermined value, or the first to fourth pulse wave signals have levels less than or equal to a third predetermined value.

In this instance, the controller 170 may perform a control operation to output a message for adjustment of positions of the first pulse wave sensor 130, the second pulse wave sensor 135 and the pressurization unit 187 such as the audio 710 saying "Tighten the band" of FIG. 7A as an audio or a video through the audio output unit 160 or the display 180. Thus, a user may adjust a position or a size of the band worn on the wrist 700 to measure the blood pressure again. As a result, the pressurization unit 187 and the like may be positioned on the radial artery 702.

As illustrated in FIGS. 7A to 7D, when various audio messages are output, user convenience may be enhanced. Messages corresponding to FIGS. 7A to 7D may be displayed on the display unit 180.

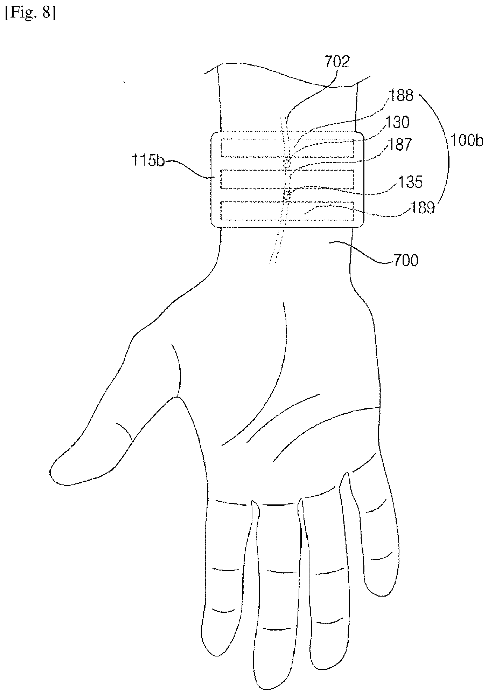

FIG. 8 illustrates an example of a hemadynamometer according to another embodiment of the present invention.

Referring to FIG. 8, the hemadynamometer of FIG. 8, denoted by reference numeral 100b, is similar to the hemadynamometer 100 of FIG. 1, and is different from the hemadynamometer 100 in that second and third pressurization units 188 and 189 are additionally provided.

The second and third pressurization units 188 and 189 are disposed opposite each other around a pressurization unit 187, a first pulse wave sensor 130 is disposed between the pressurization unit 187 and the second pressurization unit 188, and a second pulse wave sensor 135 is disposed between the pressurization unit 187 and the third pressurization unit 189.

According to this configuration, the first pulse wave sensor 130 is disposed between the pressurization unit 187 and the second pressurization unit 188, and the second pulse wave sensor 135 is disposed between the pressurization unit 187 and the third pressurization unit 189, so that the first and second pulse wave sensors 130 and 135 further stick to a wrist 700. Therefore, it is possible to more accurately measure the blood pressure at a radial artery 702.

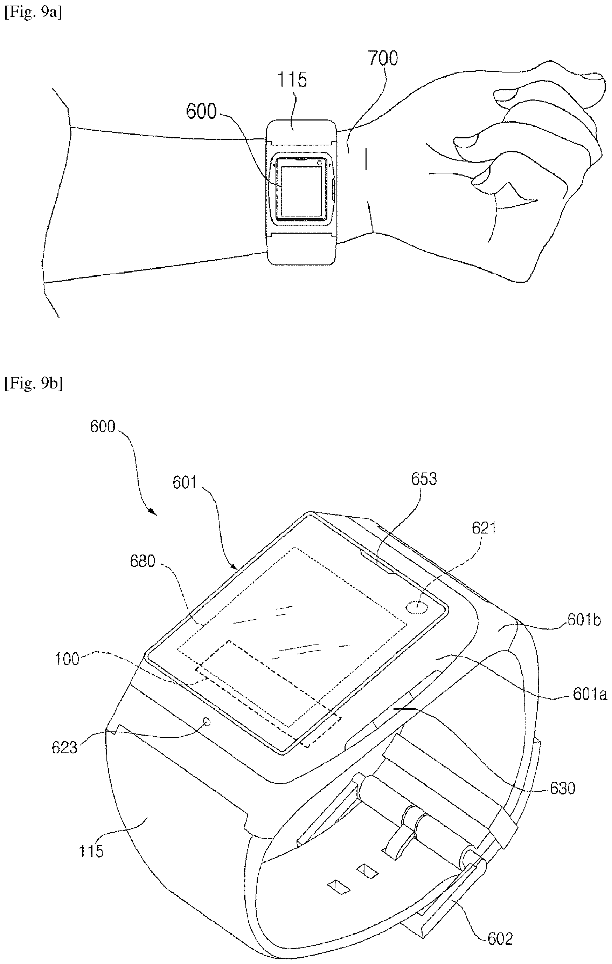

FIG. 9A illustrates that a mobile terminal equipped with the hemadynamometer 100 of FIG. 1 is worn on the wrist 700.

Referring to FIG. 9A, the wearable mobile terminal, denoted by reference numeral 600, may be a smart watch capable of displaying time information, and displaying a portion of information received by a separate smartphone through communication with the smartphone.

The wearable mobile terminal 600 of FIG. 9A may be a mobile terminal capable of independently receiving and transmitting a call separately from a smartphone.

FIG. 9B illustrates the wearable mobile terminal 600 of FIG. 9A.

Referring to FIG. 9B, the wearable mobile terminal 600 includes a main body 601 provided with the display unit 680, and a band 115 connected to the main body 601 and wearable on the wrist 700. In general, the wearable mobile terminal 600 may have a characteristic which is the same as or similar to a characteristic of the mobile terminal 100 of FIG. 1.

The main body 601 includes a case that forms an appearance. As illustrated in FIG. 9B, the case may include a first case 601a having an internal space to accommodate various electronic components and a second case 601b. The present invention is not limited thereto, and a single case may have the internal space to implement the unibody mobile terminal 600.

The wearable mobile terminal 600 may perform wireless communication, and the main body 601 may have an antenna installed to perform the wireless communication. The performance of the antenna may be extended using the case. For example, the case, which includes a conductive material, may be electrically connected to the antenna to extend a ground area or a radiation area.

The main body 601 may include the display unit 680 disposed on a front surface to output information, and the display unit 680 may be implemented as a touchscreen having a touch sensor. As illustrated in FIG. 9B, a window (not shown) of the display unit 680 may be mounted on the first case 601a to form a front surface of a terminal body together with the first case 601a.

The main body 601 may include an audio output unit 653, a camera 621, a microphone 623, a user input unit 630, and the like. When the display unit 680 is implemented as a touchscreen, the display unit 680 may function as the user input unit 630. Therefore, the main body 601 may not have a separate key.

The band 115 is worn on the wrist 700 to surround the wrist 700, and may be formed using a flexible material to be easily worn. For example, the band 115 may be made of a material such as leather, rubber, silicon, synthetic resin, or the like. In addition, the band 115 may be attached to and detached from the main body 601, and be replaced by various forms of bands according to user preference.

In addition, the band 115 may be used to extend the performance of the antenna. For example, the band may include a ground extension unit (not illustrated) electrically connected to the antenna to extend a ground area.

The band 115 may include a fastener 602. The fastener 602 may be implemented by a buckle, a hook structure for snap-fit, Velcro (trade name), or the like. The fastener 602 may include a flexible section or material. FIG. 9B illustrates an example in which the fastener 602 is implemented by a buckle.

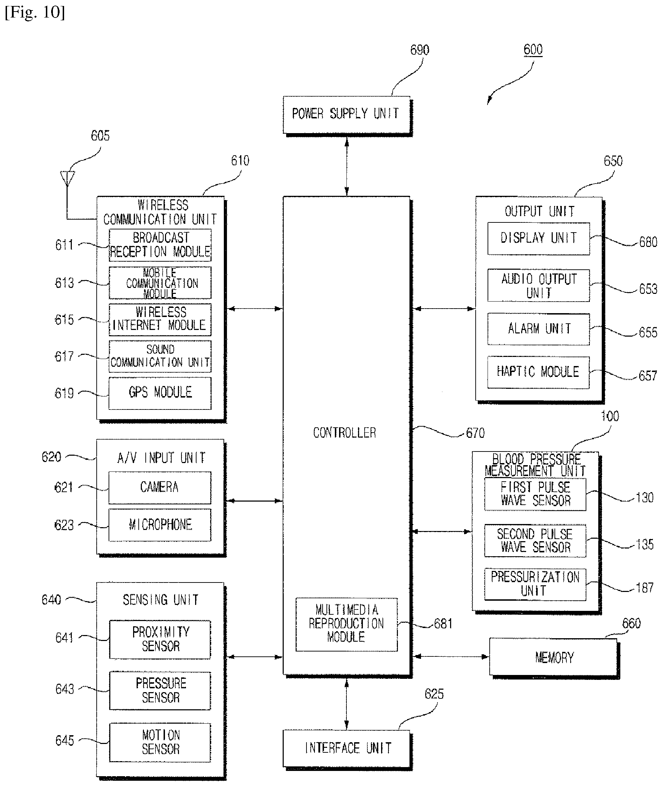

FIG. 10 shows an internal configuration of the wearable mobile terminal 600 of FIG. 9A or 9B.

Referring to FIG. 10, the mobile terminal 600 may include a wireless communication unit 610, an audio/video (A/V) input unit 620, a user input unit 630, a sensing unit 640, an output unit 650, a memory 660, an interface unit 625, a controller 670, and a power supply unit 690.

The wireless communication unit 610 may include a broadcast reception module 611, a mobile communication module 613, a wireless Internet module 615, a sound communication unit 617, a GPS module 619, and the like.

The broadcast reception module 611 may receive at least one of a broadcast signal and broadcast related information from an external broadcast management server through a broadcast channel.

The broadcast signal and/or the broadcast related information received through the broadcast reception module 611 may be stored in the memory 660.

The mobile communication module 613 transmits and receives a radio signal to and from at least one of a base station, an external terminal, and a server in a mobile communication network. Here, the radio signal may include a voice call signal, a video call signal, or various forms of data based on transmission and reception of a text/ multimedia message.

The wireless Internet module 615 refers to a module for wireless Internet access. The wireless Internet module 615 may be provided on the inside or the outside of the mobile terminal 600.

The sound communication unit 617 may perform sound communication. The sound communication unit 617 may output a sound by additionally providing predetermined information data to audio data to be output in a sound communication mode. In addition, the sound communication unit 617 may extract predetermined information data from a sound received from the outside in a sound communication mode.

The GPS module 619 may receive location information from a plurality of GPS satellites.

The A/V input unit 620 is used to input an audio or a video, and may include a camera 621, a microphone 623, and the like.

The user input unit 630 generates key input data which is input to control an operation of a terminal by a user. To this end, the user input unit 630 may include a keypad, a dome switch, a touch pad (static pressure/electrostatic), and the like. In particular, when the touch pad forms a multilayer structure with the display unit 680, the touch pad may be referred to as a touchscreen.

The sensing unit 640 may generate a sensing signal for control of an operation of the mobile terminal 600 by detecting a current state of the mobile terminal 600 such as an open or closed state of the mobile terminal 600, a position of the mobile terminal 600, contact between a user and the mobile terminal 600, and the like.

The sensing unit 640 may include a proximity sensor 641, a pressure sensor 643, a motion sensor 645, and the like. The motion sensor 645 may detect a motion, a location, and the like of the mobile terminal 600 using an acceleration sensor, a gyro sensor, a gravity sensor, and the like. In particular, the gyro sensor is a sensor for measurement of angular velocity, and may detect a rotation direction (angle) with respect to a reference direction.

The output unit 650 may include the display unit 680, an audio output unit 653, an alarm unit 655, a haptic module 657, and the like.

The display unit 680 displays and outputs information processed by the mobile terminal 600. For example, information received from the wireless communication unit 610 is displayed.

As described in the foregoing, when the display unit 680 and the touch pad form a multilayer structure, and are configured as a touchscreen, the display unit 680 may be used as an input device capable of inputting information by user touch in addition to an output device.

The audio output unit 653 outputs audio data received from the wireless communication unit 610 or stored in the memory 660. The audio output unit 653 may include a speaker, a buzzer, and the like.

With regard to the present embodiment, the audio output unit 653 may output information related to a measured blood pressure as an audio. Alternatively, the audio output unit 653 may output a message for adjustment of positions of a first pulse wave sensor 130, a second pulse wave sensor 135 and a pressurization unit 187 as an audio, or output a remeasurement notification message as an audio.

The audio output unit 653 may output information necessary for an operation of the hemadynamometer 100 as an audio.

The alarm unit 655 outputs a signal for notification of event occurrence of the mobile terminal 600. For example, a signal may be output as a vibration.

The haptic module 657 generates various tactile effects perceivable by a user. A vibration effect is a representative example of a tactile effect generated by the haptic module 657.

The memory 660 may store a program for processing and control of the controller 670, and perform a function of temporarily storing input or output data (for example, a phone book, a message, a static image, a moving image, and the like).

The interface unit 625 functions as an interface with all external devices connected to the mobile terminal 600. The interface unit 625 may receive data or power from an external device to deliver the data or power to each component in the mobile terminal 600, and allow data in the mobile terminal 600 to be transmitted to an external device.

The hemadynamometer 100 described with reference to FIGS. 1 to 7D may be included in the mobile terminal 600 as a blood pressure measurement unit 100. The blood pressure measurement unit 100 includes the first pulse wave sensor 130, the second pulse wave sensor 135 and the pressurization unit 187, which will not be further described by referring to FIGS. 1 to 7D.

The hemadynamometer 100b described with reference to FIG. 8 may be employed in the mobile terminal 600.

In general, the controller 670 controls the overall operation of the mobile terminal 600 by controlling an operation of each unit. For example, the controller 670 may perform related control and processing for a voice call, data communication, a video call, and the like. In addition, the controller 670 may include a multimedia reproduction module 681 for reproduction of multimedia. The multimedia reproduction module 681 may be configured as hardware in the controller 670, and be configured as software separately from the controller 670.

It is possible to control an operation of the blood pressure measurement unit 100 related to the present invention.

For example, the controller 670 may measure a blood pressure based on first and second pulse wave signals detected respectively by the first pulse wave sensor 130 and the second pulse wave sensor 135 before a predetermined pressure is applied to the wrist 700 by the pressurization unit 187, and third and fourth pulse wave signals detected respectively by the first pulse wave sensor 130 and the second pulse wave sensor 135

after the predetermined pressure is applied to the wrist 700 by the pressurization unit 187.

The controller 670 may separate first to fourth forward wave signals and first to fourth backward wave signals respectively from the first to fourth pulse wave signals, and measure the blood pressure based on at least one of a difference in pulse width or a difference in peak value between the first and third backward wave signals and a difference in pulse width or a difference in peak value between the second and fourth forward wave signals.

The controller 670 may measure the blood pressure in proportion to the difference in pulse width or the difference in peak value.

The controller 670 may control the display unit 680 or the audio output unit 653 to output a message for adjustment of positions of the first pulse wave sensor 130, the second pulse wave sensor 135 and the pressurization unit 187, or a remeasurement notification message when the difference in pulse width is less than or equal to a first predetermined value, the difference in peak value is less than or equal to a second predetermined value, or the first to fourth pulse wave signals have levels less than or equal to a third predetermined value.

The controller 670 may measure the blood pressure based on the first and second pulse wave signals detected respectively by the first pulse wave sensor 130 and the second pulse wave sensor 135 in a state in which a first pressure is applied to the wrist 700 by the pressurization unit 187, and the third and fourth pulse wave signals detected respectively by the first pulse wave sensor 130 and the second pulse wave sensor 135 in a state in which a second pressure is applied to the wrist 700 by the pressurization unit 187.

With regard to the present embodiment, the display unit 680 may output blood pressure information.

The display unit 680 may output a message for adjustment of positions of the first pulse wave sensor 130, the second pulse wave sensor 135 and the pressurization unit 187, or output a remeasurement notification message.

The power supply unit 690 receives external power and internal power under control of the controller 670, and supplies power necessary for an operation of each component.

The block diagram of the mobile terminal 600 shown in FIG. 10 is a block diagram for the present embodiment. The respective components of the block diagram may be combined, additionally provided, or omitted according to specifications of the mobile terminal 600 which is actually implemented. Two or more components may be combined into one component, or a component may be divided into two or more components as necessary. In addition, a function performed by each block is for describing the present embodiment, and a specific operation or device does not limit the scope of the present invention.

The configurations and schemes of the embodiments described above are not applied in a restricted manner to the hemadynamometer and the mobile terminal equipped with the hemadynamometer according to the embodiments of the present invention. All or a portion of the embodiments may be selectively combined so that the embodiments may be variously changed.

The hemadynamometer and the mobile terminal including the same according to the embodiments of the present invention may simply and accurately measure a blood pressure by including a first pulse wave sensor and a second pulse wave sensor which are disposed apart from each other, and each convert a pulse wave signal corresponding to the blood pressure into an electric signal, a pressurization unit which is disposed between the first pulse wave sensor and the second pulse wave sensor to apply a pressure to a wrist to change a diameter of a blood vessel, and a controller to measure the blood pressure based on first and second pulse wave signals detected respectively by the first pulse wave sensor and the second pulse wave sensor before a predetermined pressure is applied to the wrist by the pressurization unit, and third and fourth pulse wave signals detected respectively by the first pulse wave sensor and the second pulse wave sensor after the predetermined pressure is applied to the wrist by the pressurization unit.

In particular, it is possible to estimate a diameter of a blood vessel by detecting pulse waves at two or more positions in the blood vessel while changing the diameter of the vessel using the pressurization unit, and it is possible to measure the blood pressure based on the estimated diameter.

Therefore, it is possible to easily and accurately measure the blood pressure by applying a variation in ambient temperature or body temperature without a separate device.

In addition, the hemadynamometer may be conveniently worn on the wrist or the like, so that the first and second pulse wave sensors, the pressurization unit, and the like are attached to a band.

Although the preferred embodiments of the present invention have been disclosed for illustrative purposes, the present invention is not limited to the embodiments described above, and those skilled in the art will appreciate that various modifications are possible, without departing from the scope of the invention as disclosed in the accompanying claims.

## Claims

1. A hemadynamometer (100) comprising:

   a first pulse wave sensor (130) and a second pulse wave sensor (135) disposed apart from each other, each of the first pulse wave sensor (130) and the second pulse wave sensor (135) being configured to convert a pulse wave signal corresponding to a blood pressure into an electric signal;

   a pressurization unit (187) disposed between the first pulse wave sensor (130) and the second pulse wave sensor (135) and configured to apply a pressure to a wrist (700) to change a diameter of a blood vessel; and

   a controller (170) configured to measure the blood pressure based on first and second pulse wave signals detected respectively by the first pulse wave sensor (130) and the second pulse wave sensor (135) before a predetermined pressure is applied to the wrist by the pressurization unit (187), and third and fourth pulse wave signals detected respectively by the first pulse wave sensor (130) and the second pulse wave sensor (135) after the predetermined pressure is applied to the wrist by the pressurization unit (187),

   **characterized in that** the controller (170) is configured to separate first to fourth forward wave signals and first to fourth backward wave signals from the first to fourth pulse wave signals, and measure the blood pressure based on at least one of a difference in pulse width or a difference in peak value between the first and third backward wave signals and a difference in pulse width or a difference in peak value between the second and fourth forward wave signals.

2. The hemadynamometer (100) according to claim 1, wherein the first pulse wave sensor (130), the second pulse wave sensor (135) and the pressurization unit (187) are attached to a band (115) wearable on the wrist (700), wherein each of the first pulse wave sensor (130) and the second pulse wave sensor (135) is configured to convert a pulse wave signal detected at a radial artery in the wrist (700) into an electric signal.

3. The hemadynamometer (100) according to claim 1, wherein the controller (170) is configured to measure the blood pressure in proportion to the difference in pulse width or the difference in peak value.

4. The hemadynamometer (100) according to claim 1, wherein the first pulse wave sensor (130), the second pulse wave sensor (135) and the pressurization unit (187) are attached to a band (115) wearable on the wrist (700), wherein the controller (700) is configured to perfom a control operation to output a message for adjustment of positions of the first pulse wave sensor (130), the second pulse wave sensor (135) and the pressurization unit (187), or a remeasurement notification message when the difference in pulse width is less than or equal to a first predetermined value, the difference in peak value is less than or equal to a second predetermined value, or the first to fourth pulse wave signals have levels less than or equal to a third predetermined value.

5. The hemadynamometer (100) according to claim 1, wherein the controller (170) is configured to measure the blood pressure based on the first and second pulse wave signals detected respectively by the first pulse wave sensor (130) and the second pulse wave sensor (135) in a state in which a first pressure is transmitted to the wrist (700) by the pressurization unit (187), and the third and fourth pulse wave signals detected respectively by the first pulse wave sensor (130) and the second pulse wave sensor (135) in a state in which a second pressure is transmitted to the wrist (700) by the pressurization unit (187).

6. The hemadynamometer (100) according to claim 1, further comprising a second pressurization unit (188) and a third pressurization unit (189) disposed apart from each other around the pressurization unit (187), wherein the first pulse wave sensor (130) is disposed between the pressurization unit (187) and the second pressurization unit (188), and the second pulse wave sensor (135) is disposed between the pressurization unit (187) and the third pressurization unit (189).

7. The hemadynamometer (100) according to claim 1, further comprising an audio output unit (160) configured to output information about the measured blood pressure as an audio.

8. The hemadynamometer (100) according to claim 1, further comprising a communication unit (145) configured to transmit information about the measured blood pressure to a paired mobile terminal.

9. A mobile terminal (600) comprising:

> a communication unit (610);
> a display (660) configured to display information received from the communication unit (610); and the hemadynamometer (100) according to any of claims 1 to 5.

10. The mobile terminal (600) according to claim 9, wherein the display (660) is attached to a band (115) wearable on the wrist (700), wherein the first pulse wave sensor (130), the second pulse wave sensor (135) and the pressurization unit (187) are disposed on a rear surface of the display (660).

**Patentansprüche**

1. Hämodynamometer (100) mit :

einem ersten Pulswellensensor (130) und einem zweiten Pulswellensensor (135), die getrennt voneinander angeordnet sind, wobei sowohl der erste Pulswellensensor (130) als auch der zweite Pulswellensensor (135) dazu eingerichtet ist, ein einem Blutdruck entsprechendes Pulswellensignal in ein elektrisches Signal umzuwandeln;
einer zwischen dem ersten Pulswellensensor (130) und dem zweiten Pulswellensensor (135) angeordneten Druckbeaufschlagungseinheit (187), die dazu eingerichtet ist, einen Druck an ein Handgelenk (700) anzulegen, um einen Durchmesser eines Blutgefäßes zu ändern; und
einer Steuerung (170), die dazu eingerichtet ist, den Blutdruck zu messen basierend auf jeweils von dem ersten Pulswellensensor (134) und dem zweiten Pulswellensensor (135) erfassten ersten und zweiten Pulswellensignalen, bevor ein vorbestimmter Druck durch die Druckbeaufschlagungseinheit (187) an das Handgelenk angelegt wird, und jeweils von dem ersten Pulswellensensor (130) und dem zweiten Pulswellensensor (135) erfassten dritten und vierten Pulswellensignalen, nachdem der vorbestimmte Druck an das Handgelenk durch die Druckbeaufschlagungseinheit (187) angelegt wird,
**dadurch gekennzeichnet, dass**
die Steuerung (170) dazu eingerichtet ist, erste bis vierte Vorwärtswellensignale und erste bis vierte Rückwärtswellensignale von den ersten bis vierten Pulswellensignalen abzutrennen, und den Blutdruck basierend auf einem Unterschied in der Pulsbreite und/oder einem Unterschied des Maximalwerts zwischen dem ersten und dem dritten Rückwärtswellensignal und einem Unterschied in der Pulsbreite und/oder einem Unterschied des Maximalwerts zwischen dem zweiten und dem vierten Vorwärtswellensignal zu messen.

2. Hämodynamometer (100) nach Anspruch 1, wobei der erste Pulswellensensor (130), der zweite Pulswellensensor (135) und die Druckbeaufschlagungseinheit (187) an einem Band (115) befestigt sind, das an dem Handgelenk (700) tragbar ist, wobei sowohl der erste Pulswellensensor (130) als auch der zweite Pulswellensensor (135) dazu eingerichtet ist, ein an einer Speichenarterie im Handgelenk (700) erfasstes Pulswellensignal in ein elektrisches Signal umzuwandeln.

3. Hämodynamometer (100) nach Anspruch 1, wobei die Steuerung (170) dazu eingerichtet ist, den Blutdruck im Verhältnis zu dem Unterschied in der Pulsbreite oder dem Unterschied des Maximalwerts zu messen.

4. Hämodynamometer (100) nach Anspruch 1, wobei der erste Pulswellensensor (130), der zweite Pulswellensensor (135) und die Druckbeaufschlagungseinheit (187) an einem Band (115) befestigt sind, das an dem Handgelenk (700) tragbar ist, wobei die Steuerung (700) dazu eingerichtet ist, einen Steuervorgang auszuführen, um eine Meldung zur Einstellung von Positionen des ersten Pulswellensensors (130), des zweiten Pulswellensensors (135) und der Druckbeaufschlagungseinheit (187) auszugeben, oder eine Benachrichtigungsmeldung zum erneuten Messen, wenn der Unterschied in der Pulsbreite kleiner oder gleich einem ersten vorbestimmten Wert ist, der Unterschied des Maximalwerts kleiner oder gleich einem zweiten vorbestimmten Wert ist, oder die Pegel der ersten bis vierten Pulswellensignale kleiner oder gleich einem dritten vorbestimmten Wert sind.

5. Hämodynamometer (100) nach Anspruch 1, wobei die Steuerung (170) dazu eingerichtet ist, den Blutdruck basierend auf den jeweils von dem ersten Pulswellensensor (130) und dem zweiten Pulswellensensor (135) erfassten ersten und zweiten Pulswellensignalen zu messen, in einem Zustand, in dem ein erster Druck durch die Druckbeaufschlagungseinheit (187) auf das Handgelenk (700) übertragen wird, und den jeweils von dem ersten Pulswellensensor (130) und dem zweiten Pulswellensensor (135) erfassten dritten und vierten Pulswellensignalen, in einem Zustand, in dem ein zweiter Druck von der Druckbeaufschlagungseinheit (187) auf das Handgelenk (700) übertragen wird.

6. Hämodynamometer (100) nach Anspruch 1, ferner umfassend eine zweite Druckbeaufschlagungseinheit (188) und eine dritte Druckbeaufschlagungseinheit (189), die getrennt voneinander um die Druckbeaufschlagungseinheit (187) herum angeordnet sind, wobei der erste Pulswellensensor (130) zwischen der Druckbeaufschlagungseinheit (187) und der zweiten Druckbeaufschlagungseinheit (188) angeordnet ist, und der zweite Pulswellensensor (135) zwischen der Druckbeaufschlagungseinheit (187) und der dritten Druckbeaufschlagungseinheit (189) angeordnet ist.

7. Hämodynamometer (100) nach Anspruch 1, ferner umfassend eine Audioausgabeeinheit (160), die dazu eingerichtet ist, Informationen über den gemessenen Blutdruck als einen Ton auszugeben.

8. Hämodynamometer (100) nach Anspruch 1, ferner umfassend eine Kommunikationseinheit (145), die dazu eingerichtet ist, Informationen über den gemessenen Blutdruck an ein gekoppeltes mobiles Endgerät zu übermitteln.

9. Mobiles Endgerät (600) mit:

   einer Kommunikationseinheit (610 );
   einer Anzeige (660), die dazu eingerichtet ist, von der Kommunikationseinheit (610) erhaltene Informationen anzuzeigen;
   und dem Hämodynamometer (100) nach einem der Ansprüche 1 bis 5.

10. Mobiles Endgerät (600) nach Anspruch 9, wobei die Anzeige (660) an einem Band (115) befestigt ist, das an dem Handgelenk (700) tragbar ist, wobei der erste Pulswellensensor (130), der zweite Pulswellensensor (135) und die Druckbeaufschlagungseinheit (187) auf einer Rückseite der Anzeige (660) angeordnet sind.

**Revendications**

1. Hémodynamomètre (100) comprenant :

   un premier capteur d'onde de pouls (130) et un second capteur d'onde de pouls (135) éloignés l'un de l'autre, le premier capteur d'onde de pouls (130) et le second capteur d'onde de pouls (135) étant conçus pour convertir un signal d'onde de pouls correspondant en une pression artérielle en signal électrique ;
   une unité de mise sous pression (187) disposée entre le premier capteur d'onde de pouls (130) et le second capteur d'onde de pouls (135) et conçue pour appliquer une pression sur le poignet (700) ; et
   un organe de commande (170) conçu pour mesurer la pression artérielle sur la base des premier et second signaux d'onde de pouls détectés respectivement par le premier capteur d'onde de pouls (130) et le second capteur d'onde de pouls (135) avant qu'une pression prédéfinie ne soit appliquée sur le poignet par l'unité de mise sous pression (187), et des troisième et quatrième signaux d'onde de pouls détectés respectivement par le premier capteur d'onde de pouls (130) et le second capteur d'onde de pouls (135) après que la pression prédéfinie est appliquée sur le poignet par l'unité de mise sous pression (187),

   **caractérisé en ce que**
   l'organe de commande (170) est conçu pour séparer le premier, le deuxième, troisième et quatrième signal d'onde de pouls avant et le premier, deuxième, troisième quatrième signal arrière à partir du premier, du deuxième, du troisième , du quatrième signal d'onde de pouls, et pour mesurer la pression artérielle sur la base d'au moins une différence de largeur d'impulsion et/ou une différence de valeur crête entre le premier et le troisième signal d'onde arrière et une différence de largeur d'impulsion et/ou une différence de valeur crête entre le second et le quatrième signal d'onde avant.

2. Hémodynamomètre (100) selon la revendication 1, le premier capteur d'onde de pouls (130), le second capteur d'onde de pouls (135) et l'unité de mise sous pression (187) étant fixés à un bracelet (115) pouvant être porté au poignet (700),
le premier capteur d'onde de pouls (130) et le second capteur d'onde de pouls (135) étant conçus pour convertir un signal d'onde de pouls détecté au niveau d'une artère radiale au poignet (700) en un signal électrique.

3. Hémodynamomètre (100) selon la revendication 1, l'organe de commande (170) étant conçu pour mesurer la pression artérielle en proportion à la différence de largeur d'impulsion ou de différence de valeur crête.

4. Hémodynamomètre (100) selon la revendication 1, le premier capteur d'onde de pouls (130), le second capteur d'onde de pouls (135) et l'unité de mise sous pression (187) étant fixés à un bracelet (115) pouvant être porté au poignet (700),
l'organe de commande (700) étant conçu pour procéder à une opération de commande pour délivrer un message destiné à régler des positions du premier capteur d'onde de pouls (187) ou un message de notification de mesure lorsque la différence de largeur d'impulsion est inférieure ou égale à une première valeur prédéfinie, la différence de valeur crête étant inférieure ou égale à une deuxième valeur prédéfinie ou le premier, le deuxième, le troisième et le quatrième signal d'onde de pouls ayant des niveaux inférieurs ou égaux à une troisième valeur prédéfinie.

5. Hémodynamomètre (100) selon la revendication 1, l'organe de commande (170) étant conçu pour mesurer la pression artérielle sur la base du premier et du deuxième signal d'onde de pouls détectés respectivement par le premier capteur d'onde de pouls (130) et du second capteur d'onde de pouls (135) dans un état dans lequel une première pression est transmise au poignet (700) par l'unité de mise sous pression (187), et le troisième et le quatrième signal d'onde de pouls détectés respectivement par le premier capteur d'onde de pouls (130) et le second capteur d'onde de pouls (135) dans un état dans lequel une seconde pression est transmise au poignet (700) par l'unité de mise sous pression (187).

6. Hémodynamomètre (100) selon la revendication 1, comprenant en outre une deuxième unité de mise sous pression (187) et une troisième unité de mise sous pression (188), et le second capteur d'onde de pouls (135) étant disposé entre l'unité de mise sous pression (187) et la troisième unité de mise sous pression (189).

7. Hémodynamomètre (100) selon la revendication 1, comprenant en outre une unité de sortie audio (160) conçu pour transmettre des informations concernant la pression artérielle mesurée à un terminal mobile apparié.

8. Hémodynamomètre (100) selon la revendication 1, comprenant en outre une unité de communication (145) conçue pour transmettre des informations concernant la pression artérielle mesurée à un terminal mobile appariée.

9. Terminal mobile (100) comprenant :

   une unité de communication (610) ;
   un écran (660) conçu pour afficher des informations reçues en provenance de l'unité de communication (610) ;
   et l'hémodynamomètre (100) selon l'une quelconque des revendications 1 à 5.

10. Terminal mobile (600) selon la revendication 9, l'écran (660) étant fixé à un bracelet (115) pouvant être porté au poignet (700),
le premier capteur d'onde de pouls (130), le second capteur d'onde de pouls (135) et l'unité de mise sous pression (187) étant disposés sur une face arrière de l'écran (660).

[Fig. 1]

[Fig. 2]

[Fig. 3a]

[Fig. 3b]

[Fig. 3c]

[Fig. 4a]

[Fig. 4b]

LEVEL

420

412 414

TIME

[Fig. 4c]

412

410

414

LEVEL

TIME

BL

[Fig. 5]

100

[Fig. 6a]

[Fig. 6b]

[Fig. 6c]

[Fig. 7a]

TIGHTEN THE BAND.

[Fig. 7b]

BLOOD PRESSURE IS
BEING MEASURED.

[Fig. 7c]

BLOOD PRESSURE
IS XXXX.

[Fig. 7d]

BLOOD PRESSURE WILL
BE MEASURED AGAIN.

[Fig. 8]

[Fig. 9a]

[Fig. 9b]

[Fig. 10]

**EP 3 179 904 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102004011681 A1